# EUROPEAN PATENT APPLICATION

(11) **EP 4 342 381 A1**
(43) Date of publication of application: **27.03.2024**
(21) Application number: 22306375.1
(22) Date of filing: 20.09.2022
(51) Int. Cl.: A61B 5/372

(54) **METHOD AND DEVICE FOR ANALYZING CORTICAL ACTIVITY OF A SUBJECT**

(71) Applicant: Corstim, 34070 Montpellier (FR)
(72) Inventor: BEUTER, Anne, 34070 MONTPELLIER (FR)
(74) Representative: Icosa

(57) **Abstract**

The present invention relates to a computer-implemented method and a device for analyzing cortical activity of a subject comprising receiving electroencephalographic data of the subject comprising at least two samples (20); for each sample: generating a topographic activity map; using the topographic activity map to calculate coordinates of a maximum amplitude of the electroencephalographic activity; calculating a spatial distance between the maximum amplitude coordinates calculated for the sample and the maximum amplitude coordinates calculated for the preceding sample in the time series; comparing the calculated spatial distance to a predefined distance threshold; and identifying and defining traveling waves of cortical activity (30).

## Description

### FIELD OF INVENTION

The present invention relates to a method and a device for the analysis of physiological signal from a subject. More in details the present invention concerns the analysis of cerebral cortical activity in order to detect and provide meaningful information on traveling waves.

### BACKGROUND OF INVENTION

It is known from the early 20^{th} century that brain regions can, to some extent, be associated with specific cognitive and executive functions. This understanding of brain functions has been discussed and improved over the years, notably by the development of neuroimaging modalities and post-processing techniques. Their application in a wide variety a research fields including memory, language, cognition, action, emotion, and various medical fields. It is nowadays possible to determine the involvement of fine brain areas in specific tasks. At a larger scale, the existence of dense anatomical and functional connections has been demonstrated across the entire brain giving birth to what is now referred to as the human connectome. This connectome constitutes a specific scope of research on its own by means of big data science and graph theory approaches.

To further improve our understanding of brain functions there is the need to clarify the link between spatial brain mapping and its network organization. One potential way to achieve this deeper understanding relies on our understanding of the spatiotemporal dynamics associated with the behavior.

Decades of research in the field of image recognition and language production have enabled to describe into finer details the spatiotemporal cortical processing and to propose models of cortical function. Despite some small discrepancies across studies, one may summarize the main steps of the picture naming process as follows: Visual processing, visual recognition, semantic processing, lemma retrieval, phonological encoding, articulation programming, speech production, in parallel with self-monitoring.

Several key areas have been identified for lexical encoding (e.g., supra marginal gyrus), semantic processing (e.g., angular gyrus, anterior inferior frontal gyrus), or phonological encoding, etc. However, despite the spatial location of these areas, the mechanisms of their synchronization and/or sequential activation, their functional and/or anatomical relationship with homologous regions in the opposite hemisphere and neighbor brain areas remains unclear and variability among studies is important and multi-causal.

These last decades, several studies have shown that cortical oscillations can propagate at the surface of the human cortex forming so-called traveling waves (TW). Such waves are described as a spatiotemporally coherent evolution of either the phase or the amplitude of electrophysiological signals (sometimes referred to as phase waves and amplitude waves). Complementary studies attempted to clarify the functional role of these traveling waves. It seems, that TW per se do not carry the information from one region to another but rather contribute to the synchronization, optimization of network connectivity and to the processing of sensory inputs. One may thus hypothesize that TW indirectly participate to the efficient treatment of neural signals. In other words, they appear to play an auxiliary but important functional role in the successful unfolding of human behavior.

In this context, the present invention proposes a method to allow deeper analysis of these traveling waves in order not only to detect their presence but also to provide an easy and intuitive way of visualizing and correlating them to behavior for a user.

### SUMMARY

A computer-implemented method for analyzing cortical activity of a subject; said method comprising:
- receiving electroencephalographic data of the subject comprising at least two samples (20), said samples defining a time series of electroencephalographic signals detected from multiple electroencephalographic electrodes;
- for each sample:
   - generating a topographic activity map representing a spatial distribution of electroencephalographic activity;
   - use the topographic activity map to calculate coordinates of a maximum amplitude of the electroencephalographic activity;
   - calculate a spatial distance D(*i*-1, *i*) between the maximum amplitude coordinates calculated for the sample and the maximum amplitude coordinates calculated for the preceding sample in the time series;
   - comparing the calculated spatial distance D(*i*-1, *i*) to a predefined distance threshold;
- identifying and defining traveling waves of cortical activity (30), wherein one traveling wave is identified and defined by grouping together successive samples in the time series for which the calculated spatial distance D(i -1, i) is inferior to the predefined distance threshold and wherein two successive samples having spatial distance D(i -1, i) superior to the predefined distance threshold are not associated to the same traveling wave;
- providing as output the identified traveling waves.

The present invention advantageously allows to detect the presence of traveling waves in the electroencephalographic signals registered from a subject, which provides important information on the network connectivity and processing of sensory input.

According to other advantageous aspects of the invention, the device comprises one or more of the features described in the following embodiment, taken alone or in any possible combination.

According to one embodiment, a group of successive samples in the time series for which the calculated spatial distance D(*i*-1, *i)* is inferior to the predefined distance threshold is identified as traveling wave if it comprises at least three samples and if the maximum amplitude coordinates of the samples successive in time in said group are spatially ordered.

According to one embodiment, the predefined distance threshold is set considering a maximum velocity for a traveling wave of approximately 30 m/s.

According to one embodiment, the samples are preprocessed by filtering on a predefined wide frequency band centered on multiples frequency logarithmically spaced.

According to one embodiment, the method further comprises calculating starting points and ending points of each identified traveling wave, and identifying a first region of the different cortical areas of the subject comprising a highest density of wave starting points and a second region of the different cortical areas comprising a highest density of ending points.

According to one embodiment, the method further comprises calculating a trajectory of each identified traveling wave.

According to one embodiment, the method further comprises, using the calculated trajectories, identifying at least one third region having density of traveling waves passing through higher than a predefined threshold.

According to one embodiment, the method further comprises, using the calculated trajectories, starting points and ending points of each identified traveling wave, identifying at least one fourth region wherein the density of traveling waves passing through, starting points and ending points is inferior to a predefined threshold.

According to one embodiment, the method further comprises generating a graphical representation of the identified traveling waves through the scalp of the subject, wherein each identified traveling wave is represented by:
- two marks, a first mark representing its starting point and a second mark representing its end point;
- a segment extending from the first to the second mark following the calculated trajectory, wherein an intensity scale is used to graphically represent the time of passage of the traveling wave along the trajectory.

According to one embodiment, the graphical representation comprises at least one first circular mark centered in the first region and at least one second circular mark centered in the second region, wherein the diameter of the at least one first and second circular mark depends from the density of starting points in the first region and ending points in the second region.

Another aspect of the present invention concerns a device for analyzing cortical activity of a subject; said device comprising:
- at least one input configured to receive electroencephalographic data of the subject comprising at least two samples, said samples defining a time series of electroencephalographic signals detected from multiple electroencephalographic electrodes;
- at least one processor configured to:
   - for each sample:
      i. generating a topographic activity map representing a spatial distribution of electroencephalographic activity;
      ii. use the topographic activity map to calculate coordinates of a maximum amplitude of the electroencephalographic activity;
      iii. calculate a spatial distance D(i -1, i) between the maximum amplitude coordinates calculated for the sample and the maximum amplitude coordinates calculated for the preceding sample in the time series;
      iv. comparing the calculated spatial distance D(i -1, *i)* to a predefined distance threshold;
   - identifying and defining traveling waves of cortical activity, wherein one traveling wave is identified and defined by grouping together successive samples in the time series for which the calculated spatial distance D(i -1, *i)* is inferior to the predefined distance threshold and wherein two successive samples having spatial distance D(i -1, i) superior to the predefined distance threshold are not associated to the same traveling wave;
- at least one output configured to provide as output the identified traveling waves.

In addition, the disclosure relates to a computer program comprising software code adapted to perform a method for analyzing cortical activity of a subject compliant with any of the above execution modes when the program is executed by a processor.

The present disclosure further pertains to a non-transitory program storage device, readable by a computer, tangibly embodying a program of instructions executable by the computer to perform a method for analyzing cortical activity of a subject, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

### DEFINITIONS

In the present invention, the following terms have the following meanings:

The terms "**adapted**" and "**configured**" are used in the present disclosure as broadly encompassing initial configuration, later adaptation or complementation of the present device, or any combination thereof alike, whether effected through material or software means (including firmware).

The term "**processor**" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

The term **"traveling waves"** refers to oscillations that propagate progressively across the cortex.

### BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:
**Figure 1** is a block diagram representing schematically a first mode of a device for analyzing cortical activity of a subject.
**Figure 2** is a schematic example of how spatial ordering of the samples is verified.
**Figure 3** is a block diagram representing schematically a second mode of the device for analyzing cortical activity of a subject.
**Figure 4** is a block diagram representing schematically a third mode of the device for analyzing cortical activity of a subject.
**Figure 5** is a block diagram representing schematically a fourth mode of the device for analyzing cortical activity of a subject.
**Figure 6** is an illustrative example of graphical representation.
**Figure 7** is a flow chart showing successive steps executed with the device for analyzing cortical activity of a subject of figure 1.
**Figure 8** shows an apparatus integrating the functions of the device for analyzing cortical activity of a subject of figure 1.

On the figures, the drawings are not to scale, and identical or similar elements are designated by the same references.

### ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

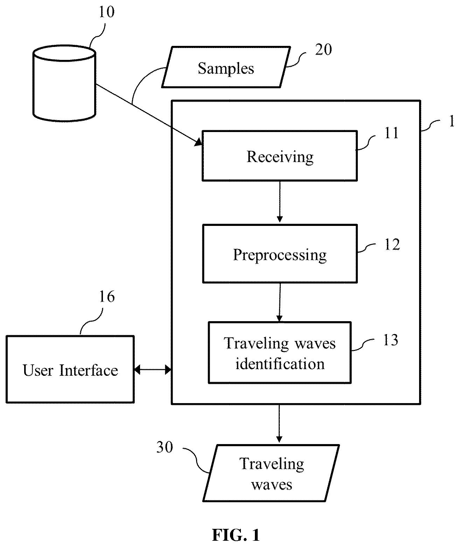

The present disclosure will be described in reference to a particular functional embodiment of a device 1 for analyzing cortical activity of a subject, as illustrated on Figure 1.

The device 1 is adapted to produce as output information concerning traveling waves that have been identified in the electroencephalographic data of the subject. Said information concerning the traveling waves may comprise coordinates of the maximum of amplitude calculated for each sample 30, the calculated trajectories 31, the starting and ending points of the trajectories taken by the traveling waves and the different regions 32 of interests corresponding to maximum or minimum densities of starting and ending points, or traversing trajectories. This information on the traveling waves provides useful insight in the interconnected network and large-scale spatiotemporal coordination of neuronal activity, for cognition and behavior, for researchers and physicians (i.e., the user).

The electroencephalographic data received as input may be obtained using electroencephalography from at least two electrodes, positioned onto predetermined areas of the scalp of the subject in order to obtain a multi-channel electroencephalographic signal. The electroencephalographic signals may be acquired by at least 4, 8, 10, 15, 16, 17, 18, 19, 20, 21, 32, 64, 128 or 256 electrodes. The electrodes may be placed on the scalp according to the 10 - 10 or 10 - 20 system, dense-array positioning or any other electrodes positioning known by the man skilled in the art. The electrodes montage may be unipolar or bipolar. The electrodes are placed accordingly to the 10 - 20 system with locations Fp1, Fp2, F7, F3, Fz, F4, F8, T3, C3, Cz, C4, T4, T5, P3, Pz, P4, T6, O1, O2, A1 and A2. For this 10 - 20 system, various types of suitable headsets or electrode systems are available for acquiring such neural signals. Examples includes, but are not limited to: Epoc headset commercially available from Emotiv, Waveguard headset commercially available from ANT Neuro, Versus headset commercially available from SenseLabs, DSI 6 headset commercially available from Wearable sensing, Xpress system commercially available from BrainProducts, Mobita system commercially available from TMSi, Porti32 system commercially available from TMSi, ActiChamp system commercially available from BrainProducts and Geodesic system commercially available from EGI. Alternatively, the electroencephalographic signals may be acquired using implanted microelectrodes arrays or intracranial electroencephalography (iEEG) or sEEG stereo-encephalography, wherein electrodes are placed directly on the surface of the brain in the epidural or subdural space to record electrical activity from the cerebral cortex. The electrical signals arising from neural activity may be as well acquired directly or indirectly using any cerebral imaging technique known by one skilled in the art. In the present description the term "electroencephalographic signals" designates all signals (local field potentials) originating from the brain independently from the techniques used to acquire them (i.e., includes the electrocorticographic signals).

The electroencephalographic signals received may be obtained with a standard recording module with sampling frequency of at least 24Hz, preferably 32 Hz, 64 Hz, 128 Hz, 250 Hz or 500 Hz, any other sampling frequency known by the man skilled in the art.

The electroencephalographic signals received may have been obtained while the subject is been presented a sensory stimulation. The sensory stimulation may be an auditory stimulation, visual stimulation and/or haptic stimulation. In one example, the subject may be stimulated using only one kind of sensory stimulation like the auditory stimulation or he/she may be stimulated simultaneously or consecutively with different kind of sensory stimulations. The use of multiple sensory modalities (auditory, visual, tactile, cross-modal) allows to achieve a high sensitivity to detect conscious perceptual processing.

Therefore, the electroencephalographic data of the subject comprising at least two samples *i,* going from 1 to *M*, each sample *i* comprising the amplitude of the *N* electrical signal register at one moment in time from the *N* electroencephalographic electrodes used for the acquisition. The samples therefore defined an ordinate time series of electrographic signals *(i.e.,* samples).

Though the presently described device 1 is versatile and provided with several functions that can be carried out alternatively or in any cumulative way, other implementations within the scope of the present disclosure include devices having only parts of the present functionalities.

The device 1 may be advantageously an apparatus, or a physical part of an apparatus, designed, configured and/or adapted for performing the mentioned functions and produce the mentioned effects or results. In alternative implementations, the device 1 is embodied as a set of apparatus or physical parts of apparatus, whether grouped in a same machine or in different, possibly remote, machines. The device 1 may e.g. have functions distributed over a cloud infrastructure and be available to users as a cloud-based service, or have remote functions accessible through an API.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1.

The device 1 comprises a module 11 for receiving the samples 20 of the electroencephalographic data that may be stored in one or more local or remote database(s) 10, for example if the samples had been previously acquired and stored, or received on-line as the acquisition of the samples 20 is in progress. When stored, the samples 20 can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

The device 1 further comprises optionally a module 12 for preprocessing the received the samples 20. The module 12 may notably be adapted to standardize the received samples 20 for sake of efficient and reliable processing. According to various configurations, the module 12 is adapted to execute only part or all of the functions described in the present description, in any possible combination, in any manner suited to the following processing stage.

In advantageous modes, the module 12 is configured for preprocessing the samples 20 so as to have the images standardized. This may enhance the efficiency of the downstream processing by the device 1. Such a standardization may be particularly useful when exploited electroencephalographic signals originating from different sources, including possibly different acquisition systems.

The module 12 may be configured to identify and remove noisy channels, and/or remove noise and/or additional artifacts using dedicated algorithms or any other method known by the skilled person. A bandpass filter may be applied, for example using a lowest cut-off frequency that may be fixed at 2 Hz, which advantageously allows to remove slow drifts that may eventually occur in the electroencephalographic data.

The module 12 may be further configured to preprocessed the samples 20 by filtering on a predefined wide frequency band centered on multiples frequency, notably logarithmically spaced. In one example, electroencephalographic data were filtered with a 1 Hz-wide band centered at nine different frequencies logarithmically spaced between 3 and 40 Hz (3, 5, 7, 9, 12, 16, 22, 30 and 40 Hz).

As shown in Figures 1, 2, 3 and 4, the device 1 further comprises a module 13 for the identification of the traveling waves. The module 13 is configured to perform a sequence of four steps on each received sample i 20. The first step consists of generating a topographic activity map for each of the received sample 20. Said topographic activity map allows to generate a continuous spatial distribution of electroencephalographic activity on the scalp of the subject from interpolation of the discrete signals obtained for a given time from the *N* acquisition electrodes placed at predefined spatial positions. Different methods, known by the person skilled in the art, may be used to obtain this topographic activity map, such as the biharmonic spline method or methods implementing linear cubic or bilinear interpolation. The topographic activity map comprises at least one maximum of the amplitude of the registered electroencephalographic signal.

The second step implemented by the module 13 comprises using the generated topographic activity map to calculate the coordinates, on the scalp of the subject, of the maximum amplitude of the electroencephalographic activity for the sample under analysis.

The third step implemented by the module 13 is configured to calculate the spatial distance D(*i*-1, *i*) on the scalp between the coordinates of the maximum amplitude for the sample *i*-1 and the coordinates of the maximum amplitude for the sample *i* (i.e., preceding sample in the time series).

The module 13 is then configured to perform the fourth step consisting in comparing the calculated spatial distance D(*i*-1, *i*) to a predefined distance threshold. Advantageously, the predefined distance threshold is set considering a maximum velocity for a traveling wave which depends on the frequency of the traveling wave detected. Said maximum velocity may vary, according to the frequency, between approximatively 1 and 10 m/s. In some examples, a maximum velocity up to approximately 30 m/s may be considered. Therefore, the predefined distance threshold may be dependent from the frequency of the traveling wave under detection. This value may be derived from literature and updated according to the evolution of the knowledge concerning the traveling waves. Therefore, two consecutives' samples (*i*-1, *i*) in time are likely to belong to one same traveling wave if the distance between the corresponding maximum amplitude coordinates may be traveled with a speed equal or inferior to 30 m/s (in the *Δt* separating the two consecutives' samples). Inversely, if the distance between two consecutives' samples (*i*-1, *i*) is too large (i.e., superior to the predefined threshold) it is likely that samples (*i*-1, *i*) are not associated to one same traveling wave. In this case, the sample (*i*-1) may be the last sample registered for one traveling wave (i.e., the end of one traveling wave) and the sample *i* may be the first sample of a new traveling wave (i.e., the start of one traveling wave). It is also possible that the signals of the sample (*i*-1) and/or *i* are/is generated from noise and/or artifacts and therefore should be excluded (i.e., not associated to a traveling wave).

The module 13 is then configured to identifying and defining traveling waves of cortical activity using the results obtained from the steps described hereabove on each sample 20. In other words, these results are used to attribute each sample 20 to one traveling wave *j* or a successive (*j*+1), or to discard the sample as not generated from a traveling wave. Some criteria have to be verified in order to determine the origin of the samples 20. As mention above, the first criterion to verify is that two consecutives' samples (*i*-1, *i)* in time are likely to belong to one same traveling wave if the distance between the corresponding maximum amplitude coordinates may be traveled with a predefined speed, *i.e*., equal or inferior to 30 m/s. Therefore, the module 13 is configured to group together all successive samples 20 in the time series for which the calculated spatial distance D(*i* -1, *i*) is inferior to the predefined distance threshold. When two successive samples (*i*-1) and (i) do not verify this first criteria, they are not associated to one same wave. Notably if sample (*i*-1) was associated, with its preceding samples 20, to one traveling wave then this sample may be considered as the end of the traveling wave. One sample 20 may be associated not grouped up with other samples if the distance from the preceding and successive samples in the time series are too distant. In the description we will refer to group also for this case of one isolate sample.

As consequence, after verifying this first criteria at least one group of sample(s) will be defined. Generally, when a large number of samples are analyzed, multiple groups of samples will be defined allowing to identify the traveling waves. After this step, each group of samples may be considered as likely associated to one traveling wave.

A second criterion may be further verified by the module 13 to reduce the risk of misattributing one group of samples to one traveling wave when the sample(s) in the group originated from a different phenomenon. Said second criterion consists in verifying that the number of sample(s) in one group is equal or superior to three. This criterion advantageously allows to filter out isolated sample (groups of 1 sample) that may, for example, originated from artefacts or noise, or groups of two samples. The person skilled in the art will know how to set the correct minimum number of samples in a group, for example according to the quality of the electroencephalographic data or other factors.

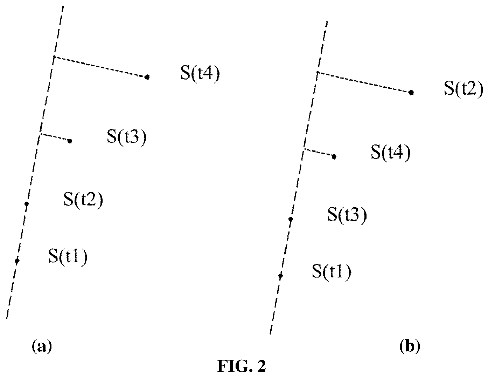

A third criterion that may be verified by module 13 to further reduce the probability of erroneously associating a group of samples to a traveling wave is that of verifying the spatial ordering of the coordinates of the amplitude maximum of the samples of one group. Figure 2a and 2b shows two examples of group of samples, that from same spatial localization of the coordinates of the maximum of amplitude of the samples of the group (i.e., not timing consideration) will be attributed to a traveling wave (Fig. 2a) and will be inversely discarded (Fig. 2b). Indeed, it can be easily seen that the point S(t2), second in the time order is not spatially ordered with respect to the other points of the group. In order to verify this spatio-temporal ordering of the maximum amplitude coordinates of the samples in one group it is possible to consider a line passing through the coordinates of two successive samples in time, e.g., first S(t1) and second S(t2) coordinates, and verify that the projection of all the coordinates of the samples in the group are ordinated on said line according to their timing. As it can be observed, the projections of the four coordinates in figure 2a are ordinated with the time while in figure 2b the projection of the third coordinate in time does not follow the projection of the second coordinate being instead placed between the projection of the first and fourth coordinates. Other method of verifying this spatio-temporal ordering of the samples may be implemented.

According to the first embodiment of the present invention represented in figure 1, the device 1 is configured to output the identified traveling waves 30, i.e., the groups of samples that have been identified by the module 13 as traveling waves.

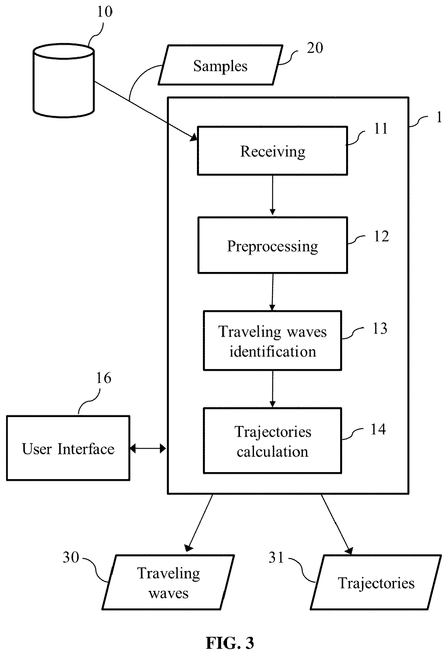

Figure 3 represent a second embodiment of the invention, according to which the device 1 further comprises a module 14 configured to calculate the trajectories of the identified traveling waves (i.e., identified by one group of samples). The trajectory of one traveling wave may be considered to pass through the maximum amplitude coordinates calculated for the samples identified as being part of said traveling wave. In one example, these coordinates may be used to define, by interpolation, a continuous trajectory starting from the coordinates of the first sample (in time) of the group and ending at the latest coordinates (i.e., latest sample in time), and which passes through all the coordinates in the group.

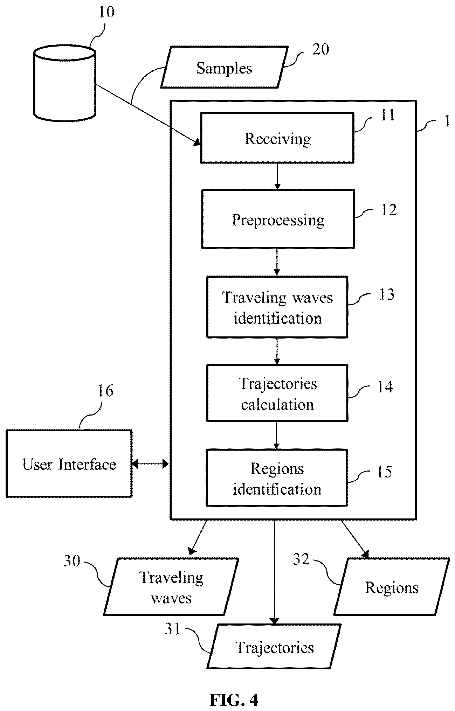

A third embodiment of the invention is represented in Figure 4, wherein the device 1 further comprises a module 15 configured to identify at least one region of interest on the scalp of the subject. The module 15 may be configured to calculate the starting point and ending point of each traveling wave. As explained above, for each group of samples associated to one traveling wave, the oldest sample of the group is associated to the origin of the traveling wave, therefore the maximum amplitude coordinates of this oldest sample may be considered as the starting point of the traveling wave. In the same way, the latest sample of the group is associated to the end of the traveling wave, therefore the maximum amplitude coordinates of this latest sample may be considered as the ending point of the traveling wave.

The module 15 may be further configured to use the information of the starting and ending points of multiple waves in order to identify a first region 32a of the scalp of the subject comprising the highest density of wave starting points and a second region 32b of the scalp comprising the highest density of ending points. The module 15 may be also configured to identify at least one first and at least one second region having density of, respectively, starting and ending points higher than a predefined threshold. Advantageously, this information concerning the traveling waves allows the user to know which region of the scalp of the subject produces the largest amount of traveling waves and where they are mainly directed.

The module 5 may be as well configured to use the calculated trajectories of the traveling waves to identify at least one third region 32c having density of traveling waves passing through higher than a predefined threshold. The third region may be calculated, as well, as the region having the highest density of traveling waves passing through.

The module 5 may be further configured to identify at least one fourth region 32d of the scalp of the subject having density of starting and end point and/or trajectories passing through inferior to a predefined threshold. This at least one fourth region advantageously allows to identify region(s) associated to low cortical activity.

This further information concerning the traveling waves (i.e., first, second, third and fourth regions) may then be provided as output from the device 1 to the user.

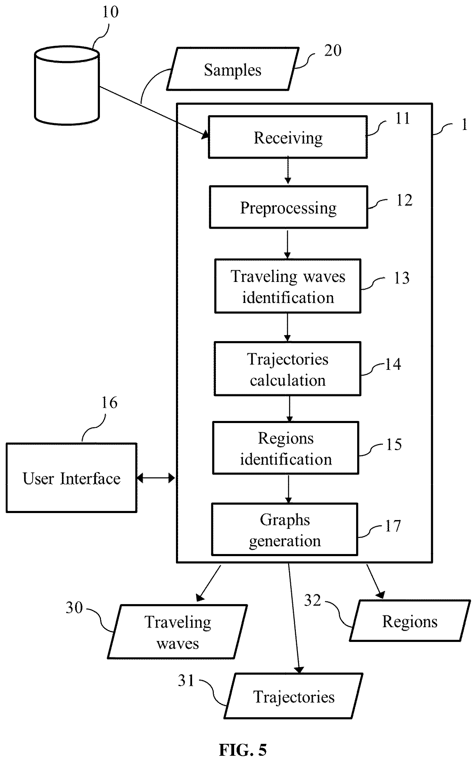

The device 1 may further comprise a module 17 for generating a graphical representation of the behavior and distribution of the traveling waves through the scalp of the subject, according to Figure 5. Said graphical representation may represent one or more identified traveling waves.

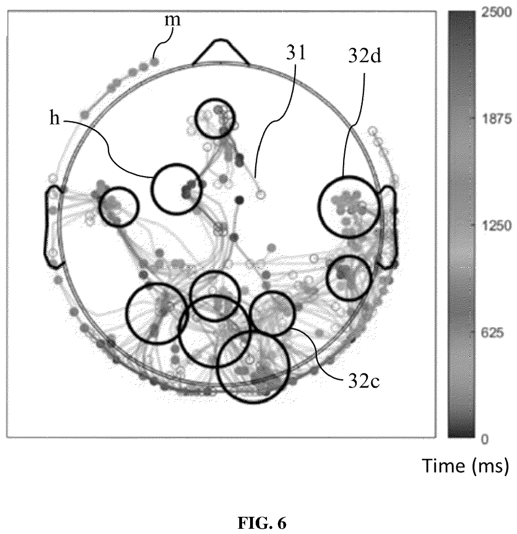

Each identified traveling wave may be represented in the graphical representation by at least two marks. The first mark representing its starting point and the second mark representing its end point. These first and second marks m are visible in Figure 6 where multiple traveling waves are drawn on a schematic representation of the scalp of the subject. One traveling wave is further represented by a segment extending from the first to the second mark following the calculated trajectory 31.

Moreover, in order to advantageously visualize the direction of propagation of the traveling wave, an intensity scale may be used to graphically represent the time of passage of the traveling wave along the trajectory 31. This intensity scale may be a color scale (e.g., grayscale or other color space). Alternatively, the intensity scale representing time may be illustrated by the thickness of the drawn trajectory, a dashed line with varying length of segments or any other visual representation suitable known by the person skilled in the art.

The graphical representation may as well comprise at least one of the following: (1) at least one first circular mark centered in the first region 32a wherein the diameter of this first circular mark depends from the density of starting points in the first region; (2) at least one second circular mark centered in the second region 32b, wherein the diameter of said second circular mark depends from the density of ending points in the second region, and/or (3) at least one third circular mark centered in one fourth region 32d wherein the diameter of this third circular mark depends from the density of traveling waves passing through, and starting points and ending points in the region. Said circular marks h are illustrated in figure 6.

This graphical representation advantageously provides a direct and meaningful visual information on the circulation of the traveling waves in the brain of a subject to be used by the user monitoring the health status of a subject, for research and/or for diagnostic.

The device 1 is interacting with a user interface 16, via which information can be entered and retrieved by a user. The user interface 16 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

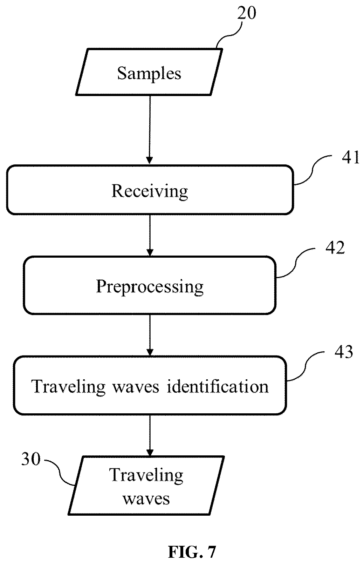

In its automatic actions, the device 1 may for example execute the following process (Figure 7):
- receiving electroencephalographic data of the subject (step 41),
- preprocessing the electroencephalographic data 21 (step 42),
- identifying and defining traveling waves 30 of cortical activity (step 43), and optionally the following steps (not represented)
- calculating trajectories 31, starting points and ending points of the traveling waves and regions of interest 32;
- generating a graphical representation of trajectories 31, starting points and ending points of the traveling waves and regions of interest 32.

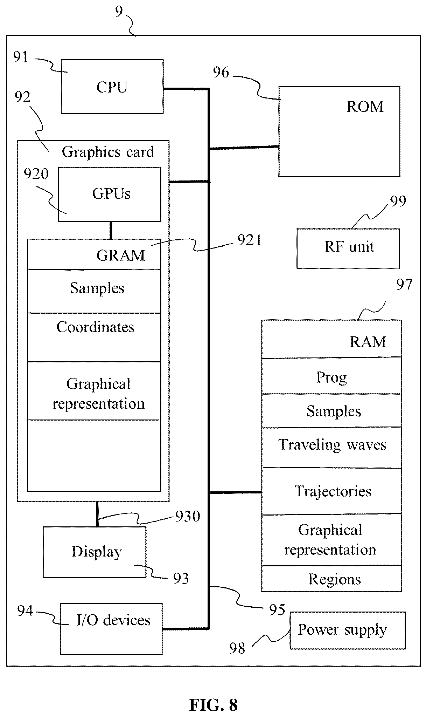

A particular apparatus 9, visible on Figure 8, is embodying the device 1 described above. It corresponds for example to a workstation, a laptop, a tablet, a smartphone, or a head-mounted display (HMD).

That apparatus 9 is suited for analyzing cortical activity of a subject. It comprises the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to signals processing, due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card, for example for obtaining the graphical representation related to the distribution of the trajectories 31, starting points and ending points of the traveling waves and regions of interest 32. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or simpler visualization implementations.

## Claims

1. A computer-implemented method for analyzing cortical activity of a subject; said method comprising:
- receiving electroencephalographic data of the subject comprising at least two samples (20), said samples defining a time series of electroencephalographic signals detected from multiple electroencephalographic electrodes;
- for each sample:
o generating a topographic activity map representing a spatial distribution of electroencephalographic activity;
o use the topographic activity map to calculate coordinates of a maximum amplitude of the electroencephalographic activity;
o calculate a spatial distance D(*i*-1, *i*) between the maximum amplitude coordinates calculated for the sample and the maximum amplitude coordinates calculated for the preceding sample in the time series;
o comparing the calculated spatial distance D(*i*-1, *i)* to a predefined distance threshold;
- identifying and defining traveling waves of cortical activity (30), wherein one traveling wave is identified and defined by grouping together successive samples in the time series for which the calculated spatial distance D(*i* -1, *i*) is inferior to the predefined distance threshold and wherein two successive samples having spatial distance D(*i* -1, *i*) superior to the predefined distance threshold are not associated to the same traveling wave;
- providing as output the identified traveling waves (30).

2. The method according to claim **1**, wherein a group of successive samples in the time series for which the calculated spatial distance D(*i*-1, *i)* is inferior to the predefined distance threshold is identified as traveling wave if it comprises at least three samples and if the maximum amplitude coordinates of the samples successive in time in said group are spatially ordered.

3. The method according to either one of claim **1** or **2**, wherein the predefined distance threshold is set considering a maximum velocity for a traveling wave of approximately 30 m/s.

4. The method according to any one of claims **1** to **3**, wherein the samples (20) are preprocessed by filtering on a predefined wide frequency band centered on multiples frequency logarithmically spaced.

5. The method according to any one of claims **1** to **4**, further comprising calculating starting points and ending points of each identified traveling wave, and identifying a first region (32a) of the different cortical areas of the subject comprising a highest density of wave starting points and a second region (32b) of the different cortical areas comprising a highest density of ending points.

6. The method according to any one of claims **1** to **5**, further comprising calculating a trajectory (31) of each identified traveling wave.

7. The method according to claim **6**, further comprising, using the calculated trajectories (31), identifying at least one third region (32c) having density of traveling waves passing through higher than a predefined threshold.

8. The method according to claim **6**, further comprising, using the calculated trajectories (31), starting points and ending points of each identified traveling wave, identifying at least one fourth region (32d) wherein the density of traveling waves passing through, starting points and ending points is inferior to a predefined threshold.

9. The method according to either one of claims **7** or **8**, further comprising generating a graphical representation of the identified traveling waves through the scalp of the subject, wherein each identified traveling wave is represented by:
- two marks, a first mark representing its starting point and a second mark representing its end point;
- a segment extending from the first to the second mark following the calculated trajectory (31), wherein an intensity scale is used to graphically represent the time of passage of the traveling wave along the trajectory (31).

10. The method according to claim **9**, wherein the graphical representation comprises at least one first circular mark centered in the first region (32a) and at least one second circular mark centered in the second region (32b), wherein the diameter of the at least one first and second circular mark depends from the density of starting points in the first region and ending points in the second region.

11. A device for analyzing cortical activity of a subject; said device comprising:
- at least one input configured to receive electroencephalographic data of the subject comprising at least two samples, said samples defining a time series of electroencephalographic signals detected from multiple electroencephalographic electrodes;
- at least one processor configured to:
o for each sample:
▪ generating a topographic activity map representing a spatial distribution of electroencephalographic activity;
▪ use the topographic activity map to calculate coordinates of a maximum amplitude of the electroencephalographic activity;
▪ calculate a spatial distance D(*i* -1, *i*) between the maximum amplitude coordinates calculated for the sample and the maximum amplitude coordinates calculated for the preceding sample in the time series;
▪ comparing the calculated spatial distance D(*i* -1, *i)* to a predefined distance threshold;
o identifying and defining traveling waves of cortical activity (30), wherein one traveling wave is identified and defined by grouping together successive samples in the time series for which the calculated spatial distance D(*i* -1, *i)* is inferior to the predefined distance threshold and wherein two successive samples having spatial distance D(*i* -1, *i*) superior to the predefined distance threshold are not associated to the same traveling wave;
- at least one output configured to provide as output the identified traveling waves (30).

12. A computer program comprising instructions which, when the program is executed by a computer, cause the computer to carry out the steps of a method for analyzing cortical activity of a subject according to any one of claims **1** to **10**.

13. A computer-readable storage medium comprising instructions which, when executed by a computer, cause the computer to carry out a method for analyzing cortical activity of a subject according to any one of claims **1** to **10**.
